# FASCICULE DE BREVET EUROPEEN

(11) **EP 0 792 633 B1**
(45) Date de publication et mention de la délivrance du brevet: **20.12.2000**
(21) Numéro de dépôt: 97400227.1
(22) Date de dépôt: 31.01.1997
(51) Int. Cl.: A61K 7/035, A61K 7/48

(54) **Utilisation d'un ester dans une composition liante des poudres et composition anhydre sous forme de poudre compacte**
Verwendung von einem Ester als Bindemittel für Pulverzusammensetzungen und wasserfreie Kompaktpulver
Use of an ester as a binder in powder compositions and anhydrous pressed power composition

(30) Priorité: 01.03.1996 FR 9602627
(43) Date de publication de la demande: 03.09.1997
(73) Titulaire: L'OREAL, 75008 Paris (FR)
(72) Inventeur: Bodelin-Lecomte, Sophie, 92170 Vanves (FR); Defossez, Béatrice, 75020 Paris (FR)
(74) Mandataire: Dodin, Catherine

(56) Documents cités:
- EP-A- 0 036 199
- EP-A- 0 665 008
- US-A- 5 063 050

## Description

La présente invention concerne l'utilisation de certains esters comme liant dans des compositions sous formes de poudres, ainsi qu'une composition sous forme de poudre comprenant comme liant au moins un ester sélectionné. La composition selon l'invention peut être utilisée dans les domaines cosmétiques, dermatologiques, pharmaceutiques et hygiéniques.

Dans le domaine des compositions cosmétiques poudreuses, sous forme de poudres compactes ou libres pour le maquillage du visage ou encore des poudres corporelles, il est connu d'utiliser, d'une part, une phase particulaire comportant notamment des pigments et des charges et, d'autre part, une phase grasse à titre de liant comprenant des corps gras, destinée à conférer au produit fini une certaine densité, à donner une certaine cohésion aux particules minérales et/ou organiques de la phase particulaire, à donner une douceur et une propriété émolliente au produit de maquillage et à favoriser son adhérence sur la peau.

Il est connu d'utiliser comme liant, des huiles d'origine pétrolière ou animale, ou bien encore des huiles de silicones, comme décrit dans le brevet US-A-5 023 075, ou des huiles fluorées comme décrit dans la demande EP-A-469 602. Des liants de poudres compactées à base d'esters d'acide gras à longue chaîne, tel que le stéarate d'isocétyle, sont également décrits dans le brevet US-A-5063050.

L'élaboration de telles compositions soulève toutefois de nombreuses difficultés car la composition finale doit être suffisamment homogène et compacte pour présenter une bonne aptitude au prélèvement et pour éviter par ailleurs une fragmentation pouvant être provoquée notamment par des chocs.
De plus, pour permettre l'application d'un produit homogène, il est nécessaire que les particules solides soient bien dispersées. Or, les liants couramment utilisés ne permettent pas d'obtenir des dispersions de poudres satisfaisantes.

La présente invention a pour but de pallier les inconvénients de l'art antérieur et de proposer une composition sous forme de poudre compactée ou coulée présentant une bonne dispersion des particules solides. De plus, lorsque la composition est compacte, celle-ci présente une bonne cohésion : la composition ainsi obtenue est plus solide, plus résistante aux chocs et ne se fragmente pas aisément.
Il est connu de EP 0 036 199 une composition sous forme de poudre comprenant des pigments enrobés fibroïne et des pigments enrobés par une matière huileuse. Le demandeur a découvert de façon inattendue et surprenante que la dispersion des poudres pouvaient être améliorées en utilisant des esters particuliers.

L'invention a donc pour objet l'utilisation d'une composition liante contenant :
- (i) au moins 20 % en poids, par rapport au poids total de la composition liante, d'au moins un ester liquide à température ambiante comprenant au moins deux chaînes hydrocarbonées, chaque chaîne hydrocarbonée comportant, indépendamment, au moins 10 atomes de carbone, ledit ester ne comportant pas de groupement hydroxyle et ayant une mouillabilité allant de 30 secondes à 10 minutes,
- (ii) l'éventuel complément à 100 % en poids, par rapport au poids total de la composition liante, d'au moins un corps gras compatible avec ledit ester,
   dans une composition anhydre sous forme de poudre comprenant au moins un composé pulvérulent, en tant qu'agent d'aide à la dispersion de ladite poudre.
   L'invention a également pour objet l'utilisation de la composition liante telle que décrite précédemment dans une composition anhydre sous forme de poudre compacte, afin d'améliorer la solidité et/ou la résistance notamment aux chocs de ladite composition anhydre.
   Un autre objet de l'invention est une composition anhydre sous forme de poudre compacte, notamment compactée ou coulée, comprenant un liant et un composé pulvérulent, caractérisée par le fait que le liant comprend au moins 90 % en poids, par rapport au poids total de liant de la composition, d'une composition liante contenant :
- (i) au moins 20 % en poids, par rapport au poids total de la composition liante, d'au moins un ester liquide à température ambiante comprenant au moins deux chaînes hydrocarbonées, chaque chaîne hydrocarbonée comportant, indépendamment, au moins 10 atomes de carbone, ledit ester ne comportant pas de groupement hydroxyle et ayant une mouillabilité allant de 5 minutes à 10 minutes,
- (ii) l'éventuel complément à 100 % en poids, par rapport au poids total de la composition liante, d'au moins un corps gras compatible avec ledit ester.

Avantageusement, la composition liante peut contenir au moins 30 %, et de préférence au moins 45 %, en poids dudit ester, ou d'un mélange, par rapport au poids total de la composition liante. Selon une variante de réalisation de l'invention, la composition liante peut consister uniquement en ledit ester.
On entend par ester liquide dans la présente description un ester apte à s'écouler à température ambiante.
La mouillabilité de l'ester selon l'invention est déterminée selon le protocole décrit avant les exemples d'illustration. Elle est comprise entre 30 secondes et 10 minutes et de préférence entre 50 secondes et 9 minutes.
De manière préférée, lorsque la composition selon l'invention se présente sous forme de poudre compacte, la mouillabilité de l'ester peut aller avantageusement de 5 minutes à 10 minutes, et préférentiellement de 8 à 9 minutes. On obtient alors de très bons résultats de cohésion des poudres compactes avec ces esters.
Selon l'invention, les chaînes hydrocarbonées de l'ester peuvent comporter, indépendamment, de préférence de 12 à 40 atomes de carbone.
Avantageusement, l'ester peut être choisi parmi le tri-isostéarate de glycéryle, le stéarate d'isocétyle, le stéarate de stéaroyl octyldécyle, le laurate d'isodécyle, ou leur mélange. De manière préférée, on peut utiliser le tri-isostéarate de glycéryle, notamment lorsque la composition est sous forme de poudre compacte pour obtenir de bonne propriétés de cohésion.
Bien entendu, la composition liante selon l'invention peut être constituée en totalité d'un ou de plusieurs esters en mélange tels que définis précédemment.

Par corps gras compatibles avec l'ester, on entend dans la présente description tout composé insoluble dans l'eau dont le mélange avec l'ester est limpide et homogène après conservation pendant 2 mois à 45°C, c'est-à-dire que le mélange ne forme qu'une seule phase sans apparition de trouble.
Avantageusement, le corps gras compatible a une viscosité, à 25°C, de 10⁻⁶ m²/s à 10⁻³ m²/s.

De préférence, le corps gras compatible peut être choisi parmi les silicones phénylées, les alkyl(C₆-C₃₀) diméthicones, la chaîne alkyle pouvant être interrompue par une fonction ester, les alcoxy(C₆-C₃₀) diméthicones, les poly α-oléfines, ou leur mélange.

Les silicones phénylées sont de préférence choisies parmi les huiles siliconées phénylées notamment les polyphénylméthylsiloxanes ou les phényltriméthicones, ou leurs mélanges, et en particulier les huiles siliconées phénylées répondant à la formule (I) suivante : 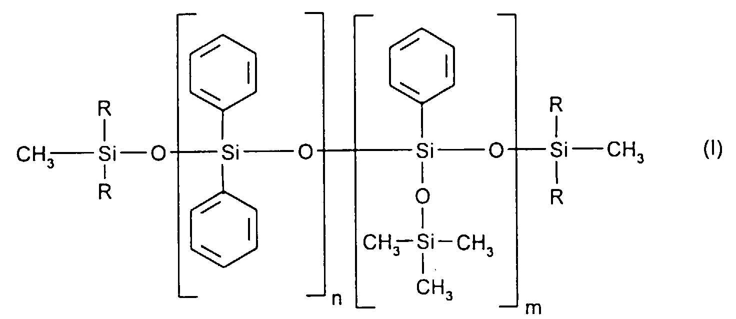 dans laquelle
. R est un radical alkyle en C1-C30, un radical aryle ou un radical aralkyle,
. n est un nombre entier compris entre 0 et 100,
. m est un nombre entier compris entre 0 et 100, sous réserve que la somme m+n est comprise entre 1 et 100.
De préférence, R est un radical méthyle, éthyle, propyle, isopropyle, décyle, dodécyle ou octadécyle, ou encore un radical phényle, tolyle, benzyle ou phénéthyle.
Parmi ces huiles phénylées, on peut citer l'huile BELSIL PDM1000 de WACKER, les huiles DC556 ou SF558 de DOW CORNING, l'huile ABIL AV8853 de GOLDSCHMIDT ou l'huile SILBIONE 70633V30 de RHONE POULENC.

Les poly-α-oléfines peuvent être en particulier :
- (i) de type polybutène, hydrogéné ou non, et de préférence polyisobutène, hydrogéné ou non. On utilise de préférence les oligomères d'isobutylène de poids moléculaire inférieur à 1000 et leurs mélange avec des polyisobutylènes de poids moléculaire supérieur à 1000 et de préférence compris entre 1000 et 15000. A titre d'exemples de poly-α-oléfines utilisables dans le cadre de la présente invention, on peut plus particulièrement mentionner les produits vendus sous le nom de PERMETHYL 99 A, 101 A , 102 A , 104 A (n=16) et 106 A (n=38) par la Société PRESPERSE Inc, ou bien encore les produits vendus sous le nom de ARLAMOL HD (n=3) par la Société ICI (n désignant le degré de polymérisation),
- (ii) de type polyisoprène. De tels produits sont vendus par exemple sous les dénominations SYNTHESQUAL par la société VEVY.
- (iii) de type polydécène, hydrogéné ou non. De tels produits sont vendus par exemple sous les dénominations ETHYLFLO par la société ETHYL CORP., et d'ARLAMOL PAO par la société ICI.
Plus particulièrement, on peut utiliser comme corps gras compatible les silicones phénylées et avantageusement les phényltriméthicones, et/ou les polydécènes.

Selon un mode préféré de réalisation de la composition selon l'invention, la composition liante comprend de 30 à 50 % dudit ester et de 50 à 70 % de corps gras compatible.

En plus de la composition liante, le liant peut comprendre, à un taux ne dépassant pas 10 % en poids du poids total de liant, d'autres corps gras couramment utilisés tels que les cires d'origine végétale, animale ou de synthèse, les huiles fluorées, la lanoline. Ces corps gras supplémentaires sont choisis de manière à ne pas modifier les propriétés avantageuses de dispersion et de cohésion conférées par la composition liante selon l'invention.
Selon un mode préféré de réalisation de la composition selon l'invention, le liant consiste en 100 % en poids de ladite composition liante.
Le liant peut être présent à raison de 1-30 %, de préférence de 3-25 % en poids, par rapport au poids total de la composition.

Les composés pulvérulents présents dans la composition peuvent être choisis parmi les pigments, les nacres et/ou les charges. Ils sont de préférence présents à raison de 70-99% en poids de la composition.

Parmi les pigments, qui peuvent être présents à raison de 0,5-80% en poids, on peut citer les pigments minéraux tels que le dioxyde de titane (rutile ou anatase) éventuellement traité en surface, les oxydes de fer noir, jaune, rouge, le violet de manganèse, le bleu outremer, le violet d'outremer, l'oxyde de chrome anhydre ou hydraté et le bleu ferrique. Les pigments organiques peuvent être choisis parmi le noir de carbone, les pigments de type D & C, et les laques à base de carmin de cochenille.
Les nacres, qui peuvent être présentes à raison de 0-50% en poids, peuvent être choisis parmi les pigments nacrés tels que le mica recouvert de pigments organiques et/ou minéraux tel que l'oxyde de titane ou l'oxychlorure de bismuth, le mica titane recouvert de pigments organiques et/ou minéraux tel que les oxydes de fer, le bleu ferrique ou l'oxyde de chrome, ainsi que les pigments nacrés à base d'oxychlorure de bismuth.
Les charges, qui peuvent être présentes à raison de 0,1-95% en poids dans la composition, peuvent être minérales ou organiques, lamellaires ou sphériques. On peut citer le talc, le mica, la silice, le kaolin, les poudres de Nylon, de poly-β-alanine et de polyéthylène, le Téflon, la lauroyl-lysine, l'amidon, le nitrure de bore, l'oxychlorure de bismuth, les poudres de polymères de tétrafluoroéthylène, les poudres de polyméthylméthacrylate, les poudres de polyuréthanne, les poudres de polystyrène, les poudres de polyester, les microsphères creuses telles que l'Expancel (Nobel Industrie), le polytrap (Dow Corning) et les microbilles de résine de silicone (Tospearls de Toshiba, par exemple), les oxydes de zinc et de titane, le carbonate de calcium précipité, le carbonate et l'hydrocarbonate de magnésium, l'hydroxyapatite, les microsphères de silice creuses (SILICA BEADS de MAPRECOS), les microcapsules de verre ou de céramique; les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition selon l'invention peut également comprendre d'autres additifs couramment utilisés, notamment dans le domaine cosmétique. Ces additifs sont choisis en fonction de l'effet souhaité pour la composition finale lors de l'application, tel que la couvrance, la transparence, la matité et/ou l'aspect satiné. On peut citer sans limitation les filtres solaires, les vitamines, les agents hydratants, les agents cicatrisants, les agents adoucissants, les agents émollients, les agents anti-acné, des parfums, des agents antiseptiques ou des agents astringents qui sont notamment utilisés dans les poudres désodorisantes ou dans les poudres pour bébés.

Les procédés de fabrication des compositions selon l'invention ne diffèrent en rien des procédés classiquement utilisés, notamment en cosmétique, et parfaitement connus de l'homme de l'art.

La composition selon l'invention se présente sous forme anhydre.
Par composition anhydre, on entend dans la présente description une composition exempte d'eau sans pour autant exclure l'eau de cristallisation des ingrédients utilisés ainsi que l'eau résultant de l'humidité de l'air ambiant qui peut être présent dans la composition, notamment pendant le stockage.

La composition compacte selon l'invention peut être notamment une poudre compactée ou bien encore une poudre coulée.
La poudre compactée peut être préparée, de manière connue, par pressage de la poudre, notamment à l'aide d'une compacteuse.
La poudre coulée peut être préparée par mise en suspension des constituants de la poudre dans un solvant (par exemple l'eau, l'hexane, l'isopropanol, l'éthanol, etc.), puis le mélange est coulé dans une coupelle, et le solvant est évaporé.
Lorsque l'on souhaite préparer une composition à base de plâtre/gypse, on peut la préparer en formant un mélange des poudres et des corps gras dans une phase aqueuse, puis couler le mélange obtenu et laisser sécher et prendre en masse.

La composition selon l'invention peut être utilisée comme fard à joues, fard à paupières, fond de teint, ou bien encore comme poudre corporelle, parfumée ou désodorisante, y compris de poudre pour les pieds.

### Protocole de détermination de la mouillabilité des esters :

On prépare une poudre ayant la composition suivante :
phase A
   - talc (15 M 00 de LUZENAC) 24,6 g
   - mica (MICA CONCORD 1000 DE SCIAMA) 23,55 g
   - oxychlorure de bismuth (PEARL-GLO UVR de ISP) 8,55 g
   - stéarate de zinc (Stéarate de zinc S de TISSCO) 3,2 g
   - polyamide-12 (ORGASOL 2002 D Nat Extra Cos de ATOCHEM) 21,4 g
   - dioxyde titane (HOMBITAN ANATASE FF PHARMA de SACHTLEBEN) 2,15 g
phase B
   - oxyde de fer noir (SICOMET noir 85 E 172 de BASF) 6,4 g
   - oxyde de fer rouge (SICOMET brun ZP 3569 de BASF) 6,95 g
   - oxyde de fer jaune (SICOMET noir 10 E 172 de BASF) 3,2 g

On pèse les constituants de la formule et on mélange efficacement pendant 15 minutes avec un mélangeur haute vitesse, jusqu'à obtention d'un mélange homogène. Puis on tamise la poudre sur un tamis de 160 µm.
On pèse exactement 2,5 g de poudre préparée que l'on introduit dans une matrice de compactage munie d'une coupelle possédant les dimensions suivantes :
longueur: 28 mm , largeur: 23 mm , hauteur: 3,5 mm.
Puis on compacte la poudre à une pression de 10⁷ Pa - 100 bars - (diamètre du piston:27 mm), à l'aide d'une compacteuse manuelle de type Kemwall, munie d'une trame de compactage, pour obtenir une dureté de 72 Shore A mesurée avec un duromètre Zwick.
Sur la poudre ainsi compactée, on dépose une goutte du liquide à tester à l'aide d'une pipette Pasteur propre. On dépose la goutte à la surface de la poudre compactée après avoir vérifié visuellement qu'aucune aspérité n'était visible en surface.

On chronomètre alors le temps nécessaire à la goutte pour s'absorber totalement à la surface du compact, l'absorption étant réalisée lors de la matification de la surface.
Le temps mesuré correspond à la valeur de mouillabilité du liquide testé.

On va maintenant donner des exemples illustrant la présente invention sans toutefois la limiter.

### Exemples comparatifs :

### 1) Exemple comparatif I

On a comparé 16 esters différents au niveau de leurs propriétés de dispersion et de cohésion de poudres compactées.

On a tout d'abord préparé une formule de base I ayant la composition suivante :

### Formule de base I :

Phase A :
   - talc 23 g
   - oxychlorure de bismuth 8 g
   - stéarate de zinc 3 g
   - mica 22 g
   - poudre de polyamide-12 20 g
   - dioxyde de titane 2 g
Phase B :
   - oxyde de fer noir 6 g
   - oxyde de fer rouge 6,5 g
   - oxyde de fer jaune 3 g
Phase C :
   - ester étudié 6,5 g

La composition de base a été préparée en mélangeant les constituants de la phase A et de la phase B puis on a ajouté la phase C (ester) goutte à goutte et on a mélangé à nouveau. Puis on a tamisé puis compacté 2 g de poudre dans une coupelle métallique possédant les dimensions suivantes :
longueur: 28 mm , largeur: 22 mm , hauteur: 3,5 mm,
à une pression de 6.10⁶ Pa - 60 bars - (diamètre du piston:27 mm), à l'aide d'une compacteuse manuelle de type Kemwall.

Puis on a évalué pour chaque ester testé la qualité de dispersion de la poudre compactée obtenue, selon les critères suivants :
- dispersion bonne : couleur uniforme sans présence de points blancs ou de taches
- dispersion moyenne : présence de nombreux petits points blancs et/ou taches à la surface du compact
- dispersion très mauvaise : taches très visibles

On a également mesuré la cohésion du compact par mesure de la perte de masse de poudre après 10 chutes normalisées d'une hauteur de 20 cm.
On a évalué la cohésion :
- soit selon la notation suivante :
   - ++: très bonne cohésion
   - +: cohésion correcte
   - 0: mauvaise cohésion
   - 00: cohésion très mauvaise
- soit en déterminant le pourcentage de perte de masse de poudre.

On a obtenu les résultats présentés dans le tableau ci-après.

On constate que les esters selon l'invention permettent d'obtenir des poudres compactées présentant une bonne dispersion et une bonne cohésion des poudres. Parmi ces esters, la composition compactée comprenant le triisostéarate de glycéryle a une meilleure cohésion que la composition compactée comprenant le stéarate d'isocétyle.

| **ESTER** | **MOUILLABILITE** | **DISPERSION** | **COHESION** |
|---|---|---|---|
| triisostéarate de glycéryle (*) | 8 min 35 sec | bonne | 0,17 % |
| stéarate d'isocétyle (*) | 53 sec | bonne | 1,40 % |
| stéarate d'octyldodécyle stéaroyle (*) | 3 min 30 sec | bonne | 0,17 % |
| laurate d'isodécyle (*) | 39 sec | bonne | non mesurée |
| palmitate d'éthyle-2 hexyle | 25 sec | moyenne | 00 |
| stéarate d'éthyle-2 hexyle | 30 sec | moyenne | 00 |
| adipate de di-isopropyle | 7 sec | moyenne | 00 |
| isononanoate d'isononyle | 9 sec | moyenne | ++ |
| palmitate d'isopropyle | 14 sec | moyenne | 00 |
| néopentanoate d'isostéaryle | 28 sec | moyenne | ++ |
| néopentanoate d'octyldodécyle | 35 sec | moyenne | + |
| octanoate de néopentyle glycol | 29 sec | moyenne | 0 |
| tétra-isostéarate de pentaérythrityle | 12 min 45 sec | très mauvaise | 00 |
| isostéarate de propylène glycol | 1 min 44 sec | très mauvaise | ++ |
| isostéarate de glycéryle | 33 min 30 sec | très mauvaise | ++ |
| diisostéarate de polyglycéryl (3 motifs de glycérol) | 1 h 30 min | très mauvaise | ++ |

| | | | |
|---|---|---|---|
| (*) ester selon l'invention | | | |

### 2) Exemple comparatif II

On a comparé trois esters au niveau de leur propriété de cohésion dans une formule compactée ayant la composition de base II suivante :

### Formule de base II :

Phase A :
   - Talc 44,2 g
Phase B:
   - oxyde de fer jaune 1,8 g
   - oxyde de fer noir 3 g
   - oxyde de chrome 1 g
Phase C1 :
   - mica oxyde de titane 40 g
Phase C2 :
   - ester étudié 10 g

La formule de base Il a été compactée dans les mêmes conditions que celles utilisées pour la formule de base I de l'exemple comparatif I. On a déterminé le pourcentage de perte de masse de poudre dans les mêmes conditions que celles décrites à l'exemple comparatif I.
On a obtenu les résultats suivants :

| **ESTER** | **MOUILLABILITE** | **COHESION** |
|---|---|---|
| tri-isostéarate de glycéryle (*) | 8 min 35 sec | 18,6 % |
| stéarate d'isocétyle | 53 sec | 21,0 % |
| stéarate d'octyldodécyle stéaroyle | 3 min 30 sec | 20,5 % |

On constate que la perte de produit est la plus faible pour la composition compactée comprenant le tri-isostéarate de glycéryle. Cet ester présente donc la meilleure propriété de cohésion.

### 3) Exemple comparatif III

On a préparé 4 compositions (A, A', B et B') compactées.
La composition A reproduit la formule de base I dans laquelle la phase C est constituée de 3,25 g de tri-isostéarate de glycéryle et de 3,25 g de polydécène.
La composition B reproduit la formule de base Il dans laquelle la phase C2 est constituée de 5 g de tri-isostéarate de glycéryle et de 5 g de polydécène.
Les compositions A' et B' sont analogues respectivement aux compositions A et B, le tri-isostéarate de glycéryle étant remplacé par la même quantité d'isostéarate d'isocétyle.

Le polydécène utilisé est vendu sous la dénomination SILKFLO 366 NF par la société AMOCO CHEMICAL.

Les compositions ont été compactées dans les mêmes conditions que celles décrites dans les exemples comparatifs I et II. On a déterminé le pourcentage de perte de masse de poudre et obtenu les résultats suivants :

| **Composition** | **Liant** | **Cohésion** |
|---|---|---|
| Composition A | tri-isostéarate de glycéryle(*)/polydécène (50/50) | 0,17 % |
| Composition A' | isostéarate d'isocétyle / polydécène (50/50) | 0,75 % |
| Composition B | tri-isostéarate de glycéryle(*)/polydécène (50/50) | 12,6 % |
| Composition B' | isostéarate d'isocétyle / polydécène (50/50) | 14 % |

On constate que pour les compositions A et B selon l'invention le pourcentage de perte de masse de poudre est plus faible que celui obtenu respectivement pour les compositions A' et B'. Ainsi, le tri-isostéarate de glycéryle, associé à un polydécène, conserve ses bonnes propriétés de cohésion.

### Exemple 1 :

On a préparé un fard à joues ayant la composition suivante :
Phase A :
   - sulfate de calcium semihydraté 24 g
   - talc 22,2 g
   - talc enrobé lauroyl lysine 12 g
   - billes de silice 8 g
   - mica 22 g
Phase B :
   - oxyde de fer rouge 3,5 g
   - oxyde de fer jaune 1 g
   - oxyde de fer noir 0,5 g
Phase C :
   - tri-isostéarate de glycéryle 3 g
   - phényl triméthylsiloxy trisiloxane (DC 556 FLUID COSMETIC de DOW CORNING) 3 g
   - polysorbate-20 0,8 g

La composition a été préparée en mélangeant les constituants de la phase A et de la phase B, puis en ajoutant la phase C goutte à goutte et en mélangeant à nouveau. Le mélange obtenu a ensuite été dispersé dans 70 parties d'eau pour 100 parties de mélange de poudre pour obtenir une pâte fluide.
La pâte a ensuite été coulée dans un moule de forme bombée.
Après avoir laisser reposé pendant 2 heures à température ambiante, le produit a été chauffé en étuve à 45 degrés pendant 12 heures. Puis on a démoulé le produit après séchage. Dans le produit sec, le sulfate de calcium se trouve sous forme de di-hydrate (eau de cristallisation).
Dans le produit obtenu, on a constaté une bonne dispersion des composés pulvérulents. La poudre est facile à déliter et présente une bonne douceur lors de l'application sur les joues.

### Exemple 2 :

On a préparé un fard à paupières compacté ayant la composition suivante :
Phase A
   - talc 63 g
   - mica enrobé silicone 8 g
   - poudre de polyéthylène 5 g
   - mica-titane 10 g
Phase B
   - oxyde de fer rouge 3 g
   - bleu d'outremer 3 g
Phase C
   - stéarate d'isocétyle 8 g

On a mélangé les constituants de la phase A et de la phase B, puis ajouté la phase C goutte à goutte et on a mélangé à nouveau. Après tamisage, on a compacté la poudre dans une coupelle métallique. On a obtenu un fard à paupières présentant une bonne dispersion des poudres et une bonne cohésion.

### Exemple 3 :

On a préparé un fard à paupières compacté ayant la composition suivante :
Phase A
   - talc 20 g
   - mica 10 g
   - oxychlorure de bismuth 8 g
   - mica-titane 40 g
Phase B
   - oxyde de chrome anhydre 6 g
   - bleu d'outremer 2 g
Phase C
   - tri-isostéarate de glycéryle 7 g
   - polydécène hydrogéné (SILKFLO 366 NF de ALBEMARLE) 7 g

On a préparé la composition selon le même mode opératoire de l'exemple 2.
On a obtenu un fard à paupières présentant une bonne dispersion des poudres et une bonne cohésion.

### Exemple 4 :

On a préparé un fard à joues compacté ayant la composition suivante :
Phase A
   - talc 67,8 g
   - mica 15 g
   - poudre de polyméthacrylate de méthyle 6 g
   - laurate de zinc 2 g
Phase B
   - violet de manganèse 1,2 g
   - oxyde de fer rouge 0,9 g
   - oxyde de fer noir 0,1 g
Phase C
   - stéarate d'isocétyle 2,1 g
   - polydécène hydrogéné 4,9 g

On a préparé la composition selon le même mode opératoire de l'exemple 2.
On a obtenu un fard à paupières présentant une bonne dispersion des poudres et une bonne cohésion.

### Exemple 5 :

On a préparé une poudre libre pour le visage ayant la composition suivante :
Phase A
   - mica 65,05 g
   - mica-titane-oxyde de fer jaune 8 g
   - poudre de polyamide-12 20 g
   - hyaluronate de sodium 0,1 g
   - stéarate de zinc 3 g
Phase B
   - oxyde de fer jaune 0,4 g
   - oxyde de fer rouge 0,3 g
   - oxyde de fer noir 0,15 g
Phase C
   - phényltriméthylsiloxy trisiloxane 1,8 g
   - tri-isostéarate de glycéryle 1,2 g

La composition a été préparée selon le même mode opératoire que l'exemple 2, la poudre n'étant pas compactée mais utilisée à l'état libre.
On constate que les composés pulvérulents sont bien dispersés et que la poudre s'applique facilement sur le visage et présente une bonne tenue.

### Exemple 6 :

On prépare une poudre compactée pour le visage ayant la composition suivante:
Phase A
   - séricite 66,8 g
   - mica 15 g
   - poudre de polyamide-12 6 g
   - dioxyde de titane 2 g
Phase B
   - oxyde de fer jaune 2,5 g
   - oxyde de fer rouge 1,2 g
   - oxyde de fer noir 0,5 g
Phase C
   - stéarate d'isocétyle 2,4 g
   - polydécène hydrogéné 2,4 g
   - phényltriméthylsiloxy trisiloxane 1,2 g

La composition a été préparée selon le même mode opératoire de l'exemple 2.
On a obtenu une poudre compactée pour le visage présentant une bonne dispersion de la poudre ainsi qu'une bonne cohésion.

### Exemple 7 :

On prépare une poudre compactée pour le visage ayant la composition suivante :
Phase A
   - talc enrobé silicone 20 g
   - mica 35 g
   - dioxyde de titane 4,5 g
   - talc 27,3 g
   - stéarate de zinc 3 g
Phase B
   - oxyde de fer jaune 1,6 g
   - oxyde de fer rouge 0,9 g
   - oxyde de fer noir 0,4 g
   - conservateurs 0,3 g
Phase C
   - laurate d'isodécyle 4,9 g
   - polyisoprène (SYNTHESQUAL de VEVY) 2,1 g

La composition a été préparée selon le même mode opératoire de l'exemple 2.
On a obtenu une poudre compactée pour le visage présentant une bonne dispersion de la poudre ainsi qu'une bonne cohésion. La poudre compactée peut être délitée à sec avec un applicateur classique (pinceau, mousse) ou bien à l'aide d'une éponge humide. On a constaté que la poudre s'applique facilement sur la peau et présente une bonne douceur.

## Revendications

1. Utilisation d'une composition liante contenant :
(i) au moins 20 % en poids, par rapport au poids total de la composition liante, d'au moins un ester liquide à température ambiante comprenant au moins deux chaînes hydrocarbonées, chaque chaîne hydrocarbonée comportant, indépendamment, au moins 10 atomes de carbone, ledit ester ne comportant pas de groupement hydroxyle et ayant une mouillabilité allant de 30 secondes à 10 minutes,
(ii) l'éventuel complément à 100 % en poids, par rapport au poids total de la composition liante, d'au moins un corps gras compatible avec ledit ester,
dans une composition anhydre sous forme de poudre comprenant au moins un composé pulvérulent, en tant qu'agent d'aide à la dispersion de ladite poudre.

2. Utilisation selon la revendication 1, afin d'améliorer la solidité et/ou la résistance notamment aux chocs d'une composition se présentant sous la forme de poudre compacte.

3. Utilisation selon l'une des revendications 1 ou 2, caractérisée par le fait que l'ester a une mouillabilité comprise entre 50 secondes et 9 minutes.

4. Utilisation selon l'une des revendications précédentes, caractérisée par le fait que l'ester a une mouillabilité comprise entre 8 minutes et 9 minutes.

5. Utilisation selon l'une quelconque des revendications précédentes, caractérisée par le fait que lesdites chaînes hydrocarbonées dudit ester comportent indépendamment de 12 à 40 atomes de carbone.

6. Utilisation selon l'une quelconque des revendications précédentes, caractérisée par le fait que l'ester est choisi parmi le tri-isostéarate de glycéryle, le stéarate d'isocétyle, le stéarate de stéaroyl octyldécyle, le laurate d'isodécyle et leurs mélanges.

7. Utilisation selon l'une quelconque des revendications précédentes, caractérisée par le fait que ledit corps gras compatible est choisi parmi les silicones phénylées, les alkyl(C₆-C₃₀) diméthicones, la chaîne alkyle pouvant être interrompue par une fonction ester, les alcoxy(C₆-C₃₀) diméthicones, les poly α-oléfines et leurs mélanges.

8. Utilisation selon l'une quelconque des revendications précédentes, caractérisée par le fait que le corps gras compatible a une viscosité, à 25°C, de 10⁻⁶ m²/s à 10⁻³ m²/s.

9. Utilisation selon l'une quelconque des revendications précédentes, caractérisée par le fait que ledit corps gras compatible est choisi parmi
- (i) les silicones phénylées de formule (I) : 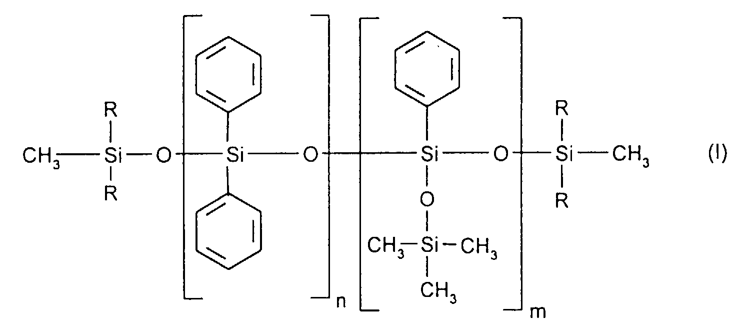 dans laquelle
. R est un radical alkyle en C1-C30, un radical aryle ou un radical aralkyle,
. n est un nombre entier compris entre 0 et 100,
. m est un nombre entier compris entre 0 et 100, sous réserve que la somme m+n est comprise entre 1 et 100,
- (ii) les poly-α-oléfines de type polybutène ou polydécène,
- (iii) leurs mélanges.

10. Utilisation selon l'une quelconque des revendications précédentes, caractérisée par le fait que la composition comprend un liant comprenant au moins 90 % en poids, par rapport au poids total du liant de la composition, de ladite composition liante.

11. Utilisation selon la revendication 10, caractérisée par le fait que le liant est présent à une teneur allant de 1 % à 30 %, et de préférence de 3 % à 25 %, en poids, par rapport au poids total de la composition.

12. Utilisation selon l'une quelconque des revendications précédentes, caractérisée par le fait que les composés pulvérulents sont choisis parmi les pigments, les charges, les nacres et leurs mélanges.

13. Utilisation selon l'une quelconque des revendications précédentes, caractérisée par le fait que les composés pulvérulents sont présents à une teneur allant de 70 % à 99 % en poids par rapport au poids total de la composition.

14. Composition anhydre sous forme de poudre compacte comprenant un liant et un composé pulvérulent, caractérisée par le fait que le liant comprend au moins 90 % en poids, par rapport au poids total de liant de la composition, d'une composition liante contenant :
- (i) au moins 20 % en poids, par rapport au poids total de la composition liante, d'au moins un ester liquide à température ambiante comprenant au moins deux chaînes hydrocarbonées, chaque chaîne hydrocarbonée comportant, indépendamment, au moins 10 atomes de carbone, ledit ester ne comportant pas de groupement hydroxyle et ayant une mouillabilité allant de 5 minutes à 10 minutes,
- (ii) l'éventuel complément à 100 % en poids, par rapport au poids total de la composition liante, d'au moins un corps gras compatible avec ledit ester.

15. Composition selon la revendication 14, caractérisée par le fait que l'ester a une mouillabilité comprise entre 8 minutes et 9 minutes.

16. Composition selon l'une quelconque des revendications 14 et 15, caractérisée par le fait que l'ester le tri-isostéarate de glycéryle.

17. Composition selon l'une quelconque des revendications 14 à 16, caractérisée par le fait que ledit corps gras compatible est choisi parmi les silicones phénylées, les alkyl(C₆-C₃₀) diméthicones, la chaîne alkyle pouvant être interrompue par une fonction ester, les alcoxy(C₆-C₃₀) diméthicones, les poly α-oléfines et leurs mélanges.

18. Composition selon l'une quelconque des revendications 14 à 17, caractérisée par le fait que ledit corps gras compatible est choisi parmi
- (i) les silicones phénylées de formule (I) : 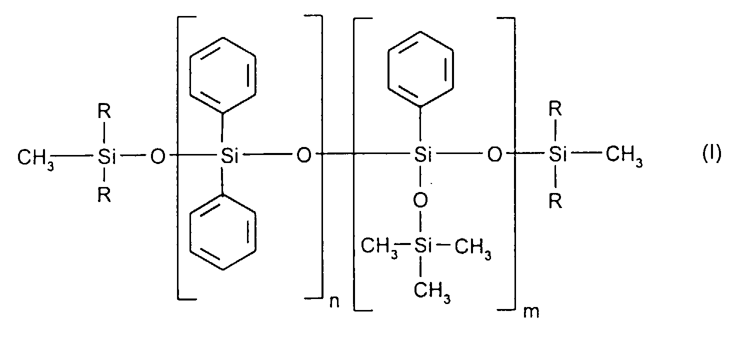 dans laquelle
. R est un radical alkyle en C1-C30, un radical aryle ou un radical aralkyle,
. n est un nombre entier compris entre 0 et 100,
. m est un nombre entier compris entre 0 et 100, sous réserve que la somme m+n est comprise entre 1 et 100,
- (ii) les poly-α-oléfines de type polybutène ou polydécène, et
- (iii) leurs mélanges.

19. Composition selon l'une quelconque des revendications 14 à 18, caractérisée par le fait que le liant est présent à une teneur allant de 1 % à 30 %, et de préférence de 3 % à 25 %, en poids, par rapport au poids total de la composition.

20. Composition selon l'une quelconque des revendications 14 à 19, caractérisée par le fait que les composés pulvérulents sont présents à une teneur allant de 70 % à 99 % en poids par rapport au poids total de la composition.

21. Composition selon l'une quelconque des revendications 14 à 20, caractérisée par le fait qu'elle se présente sous la forme de fard à joues, de fard à paupières, de fond de teint, ou de poudre corporelle.

## Patentansprüche

1. Verwendung einer bindenden Zusammensetzung, die enthält:
(i) mindestens 20 Gew.-%, bezogen auf das Gesamtgewicht der bindenden Zusammensetzung, mindestens eines bei Umgebungstemperatur flüssigen Esters, der mindestens zwei Kohlenwasserstoffketten enthält, wobei jede Kohlenwasserstoffkette unabhängig mindestens 10 Kohlenstoffatome enthält, wobei der Ester keine Hydroxygruppen enthält und eine Benetzbarkeit von 30 s bis 10 min aufweist,
(ii) einen gegebenfalls bis auf 100 Gew.-% ergänzenden Stoff, bezogen auf das Gesamtgewicht der bindenden Zusammensetzung, mindestens einer mit diesem Ester kompatiblen Fettsubstanz,
in einer wasserfreien, in Form eines Pulvers vorliegenden Zusammensetzung, die mindestens eine pulverförmige Verbindung enthält, als Hilfsmittel zum Dispergieren dieses Pulvers.

2. Verwendung nach Anspruch 1 zur Verbesserung der Festigkeit und/oder der Beständigkeit insbesondere gegen Stoßeinwirkung einer in Form eines kompakten Pulvers vorliegenden Zusammensetzung.

3. Verwendung nach einem der Ansprüche 2 und 3, dadurch gekennzeichnet, daß der Ester eine Benetzbarkeit von 50 s bis 9 min aufweist.

4. Verwendung nach einem der vorhergehenden Ansprüche, dadurch gekennzeichnet, daß der Ester eine Benetzbarkeit von 8 bis 9 min aufweist.

5. Verwendung nach einem der vorhergehenden Ansprüche, dadurch gekennzeichnet, daß die Kohlenwasserstoffketten des Esters unabhängig 12 bis 40 Kohlenstoffatome aufweisen.

6. Verwendung nach einem der vorhergehenden Ansprüche, dadurch gekennzeichnet, daß der Ester unter Glycerintriisostearat, Isocetylstearat, Octyldecylstearoylstearat, Isodecyllaurat und Gemischen dieser Ester ausgewählt wird.

7. Verwendung nach einem der vorhergehenden Ansprüche, dadurch gekennzeichnet, daß die kompatible Fettsubstanz unter phenylgruppenhaltigen Siliconen, (C₆-C₃₀)-Alkyldimethiconen, wobei die Alkylkette durch eine Estergruppe unterbrochen sein kann, (C₆-C₃₀)-Alkoxydimethiconen, Poly-α-olefinen und ihren Gemischen ausgewählt wird.

8. Verwendung nach einem der vorhergehenden Ansprüche, dadurch gekennzeichnet, daß die kompatible Fettsubstanz bei 25 °C eine Viskosität von 10⁻⁶ m²/s bis 10⁻³ m²/s aufweist.

9. Verwendung nach einem der vorhergehenden Ansprüche, dadurch gekennzeichnet, daß die kompatible Fettsubstanz ausgewählt wird unter
(i) den phenylgruppenhaltigen Siliconölen der folgenden Formel (I) 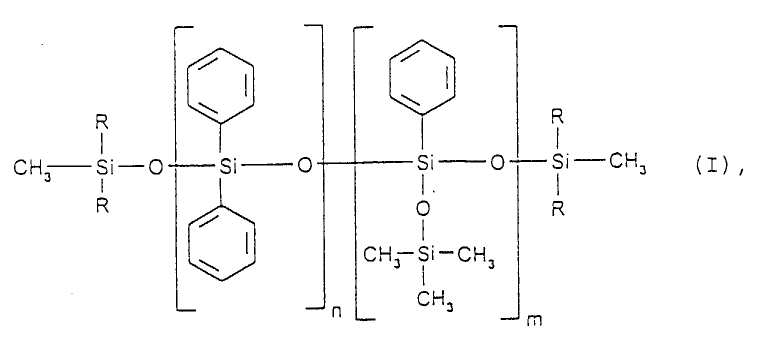 in der bedeuten:
- R C₁-C₃₀-Alkyl, Aryl oder Aralkyl,
- n eine ganze Zahl von 0 bis 100,
- m eine ganze Zahl von 0 bis 100
mit der Maßgabe, dass die Summe m+n 1 bis 100 beträgt.
(ii) Poly-α-olefine vom Polybuten-Typ und vom Polydecen-Typ und
(iii) ihren Gemischen.

10. Verwendung nach einem der vorhergehenden Ansprüche, dadurch gekennzeichnet, daß die Zusammensetzung ein Bindemittel enthält, das mindestens 90 Gew.-%, bezogen auf das Gesamtgewicht des Bindemittels der Zusammensetzung, der bindenden Zusammensetzung enthält.

11. Verwendung nach Anspruch 10, dadurch gekennzeichnet, daß das Bindemittel in einem Anteil von 1 bis 30 Gew.-% und vorzugsweise 3 bis 25 Gew.-%, bezogen auf das Gesamtgewicht der Zusammensetzung, enthalten ist.

12. Verwendung nach einem der vorhergehenden Ansprüche, dadurch gekennzeichnet, daß die pulverförmigen Verbindungen unter Pigmenten, Füllstoffen, Perlglanzmaterialien und ihren Gemischen ausgewählt werden.

13. Verwendung nach einem der vorhergehenden Ansprüche, dadurch gekennzeichnet, daß die pulverförmigen Verbindungen in einem Anteil von 70 bis 99 Gew.-%, bezogen auf das Gesamtgewicht der Zusammensetzung, enthalten sind.

14. Wasserfreie Zusammensetzung in Form eines kompakten Pulvers, die ein Bindemittel und eine pulverförmige Verbindung enthält, dadurch gekennzeichnet, daß das Bindemittel mindestens 90 Gew.-%, bezogen auf das Gesamtgewicht des Bindemittels der Zusammensetzung, einer bindenden Zusammensetzung enthält, die enthält:
(i) mindestens 20 Gew.-%, bezogen auf das Gesamtgewicht der bindenden Zusammensetzung, mindestens eines bei Umgebungstemperatur flüssigen Esters, der mindestens zwei Kohlenwasserstoffketten enthält, wobei jede Kohlenwasserstoffkette unabhängig mindestens 10 Kohlenstoffatome enthält, wobei der Ester keine Hydroxygruppen enthält und eine Benetzbarkeit von 5 min bis 10 min aufweist,
(ii) einen gegebenfalls bis auf 100 Gew.-% ergänzenden Stoff, bezogen auf das Gesamtgewicht der bindenden Zusammensetzung, mindestens einer mit diesem Ester kompatiblen Fettsubstanz.

15. Zusammensetzung nach Anspruch 14, dadurch gekennzeichnet, daß der Ester eine Benetzbarkeit von 8 bis 9 min aufweist.

16. Zusammensetzung nach einem der Ansprüche 14 und 15, dadurch gekennzeichnet, daß es sich bei dem Ester um Glycerintriisostearat handelt.

17. Zusammensetzung nach einem der Ansprüche 14 bis 16, dadurch gekennzeichnet, daß die kompatible Fettsubstanz unter phenylgruppenhaltigen Siliconen, (C₆-C₃₀)-Alkyldimethiconen, wobei die Alkylkette durch eine Estergruppe unterbrochen sein kann, (C₆-C₃₀)-Alkoxydimethiconen, Poly-α-olefinen und ihren Gemischen ausgewählt ist.

18. Zusammensetzung nach einem der Ansprüche 14 bis 17, dadurch gekennzeichnet, daß die kompatible Fettsubstanz ausgewählt ist unter
(i) den phenylgruppenhaltigen Siliconölen der folgenden Formel (I) 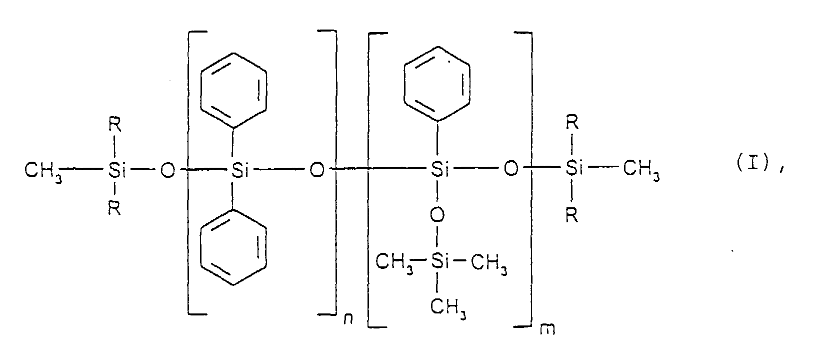 in der bedeuten:
- R C₁-C₃₀-Alkyl, Aryl oder Aralkyl,
- n eine ganze Zahl von 0 bis 100,
- m eine ganze Zahl von 0 bis 100
mit der Maßgabe, dass die Summe m+n 1 bis 100 beträgt.
(ii) Poly-α-olefinen vom Polybuten-Typ und vom Polydecen-Typ und
(iii) ihren Gemischen.

19. Zusammensetzung nach einem der Ansprüche 14 bis 18, dadurch gekennzeichnet, daß das Bindemittel in einem Anteil von 1 bis 30 Gew.-% und vorzugsweise 3 bis 25 Gew.-%, bezogen auf das Gesamtgewicht der Zusammensetzung, enthalten ist.

20. Zusammensetzung nach einem der Ansprüche 14 bis 19, dadurch gekennzeichnet, daß die pulverförmigen Verbindungen in einem Anteil von 70-99 Gew.-%, bezogen auf das Gesamtgewicht der Zusammensetzung, enthalten sind.

21. Zusammensetzung nach einem der Ansprüche 14 bis 20, dadurch gekennzeichnet, daß sie in Form eines Rouge, Lidschatten, Make-up oder als Körperpuder vorliegt.

## Claims

1. Use of a binder composition containing:
(i) at least 20% by weight, relative to the total weight of the binder composition, of at least one ester which is liquid at room temperature comprising at least two hydrocarbon chains, each hydrocarbon chain independently containing at least 10 carbon atoms, the said ester containing no hydroxyl groups and having a wettability ranging from 30 seconds to 10 minutes,
(ii) the possible difference to 100% by weight, relative to the total weight of the binder composition, of at least one fatty substance which is compatible with the said ester,
in an anhydrous composition in powder form comprising at least one pulverulent compound, as an agent for aiding the dispersion of the said powder.

2. Use according to Claim 1, in order to improve the solidity and/or strength, in particular the impact strength, of a composition which is in the form of a compact powder.

3. Use according to either of Claims 1 and 2, characterized in that the ester has a wettability of between 50 seconds and 9 minutes.

4. Use according to one of the preceding claims, characterized in that the ester has a wettability of between 8 minutes and 9 minutes.

5. Use according to any one of the preceding claims, characterized in that the said hydrocarbon chains of the said ester independently contain from 12 to 40 carbon atoms.

6. Use according to any one of the preceding claims, characterized in that the ester is chosen from glyceryl triisostearate, isocetyl stearate, stearoyloctyldecyl stearate, isodecyl laurate and mixtures thereof.

7. Use according to any one of the preceding claims, characterized in that the said compatible fatty substance is chosen from phenyl silicones, (C₆-C₃₀)alkyl dimethicones, it being possible for the alkyl chain to be interrupted by an ester function, (C₆-C₃₀)alkoxy dimethicones, poly-α-olefins and mixtures thereof.

8. Use according to any one of the preceding claims, characterized in that the compatible fatty substance has a viscosity, at 25°C, of 10⁻⁶m²/s to 10⁻³ m²/s.

9. Use according to any one of the preceding claims, characterized in that the said compatible fatty substance is chosen from
- (i) phenyl silicones of formula (I) : 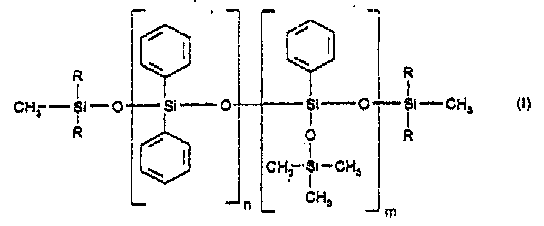 in which
. R is a C₁-C₃₀ alkyl radical, an aryl radical or an aralkyl radical,
. n is an integer between 0 and 100,
. m is an integer between 0 and 100, with the proviso that the sum m+n is between 1 and 100,
- (ii) poly-α-olefins of polybutene or polydecene type,
- (iii) mixtures thereof.

10. Use according to any one of the preceding claims, characterized in that the composition comprises a binder comprising at least 90% by weight, relative to the total weight of the binder in the composition, of the said binder composition.

11. Use according to Claim 10, characterized in that the binder is present at a content ranging from 1% to 30%, and preferably from 3% to 25%, by weight relative to the total weight of the composition.

12. Use according to any one of the preceding claims, characterized in that the pulverulent compounds are chosen from pigments, fillers, pearlescent agents and mixtures thereof.

13. Use according to any one of the preceding claims, characterized in that the pulverulent compounds are present at a content ranging from 70% to 99% by weight relative to the total weight of the composition.

14. Anhydrous composition in compact powder form comprising a binder and a pulverulent compound, characterized in that the binder comprises at least 90% by weight, relative to the total weight of binder in the composition, of a binder composition containing:
- (i) at least 20% by weight, relative to the total weight of the binder composition, of at least one ester which is liquid at room temperature comprising at least two hydrocarbon chains, each hydrocarbon chain independently containing at least 10 carbon atoms, the said ester containing no hydroxyl groups and having a wettability ranging from 5 minutes to 10 minutes,
- (ii) the possible difference to 100% by weight, relative to the total weight of the binder composition, of at least one fatty substance which is compatible with the said ester.

15. Composition according to Claim 14, characterized in that the ester has a wettability of between 8 minutes and 9 minutes.

16. Composition according to either of Claims 14 and 15, characterized in that the ester is glyceryl triisostearate.

17. Composition according to any one of Claims 14 to 16, characterized in that the said compatible fatty substance is chosen from phenyl silicones, (C₆-C₃₀)alkyl dimethicones, it being possible for the alkyl chain to be interrupted by an ester function, (C₆-C₃₀)alkoxy dimethicones, poly-α-olefins and mixtures thereof.

18. Composition according to any one of Claims 14 to 17, characterized in that the said compatible fatty substance is chosen from
- (i) phenyl silicones of formula (I) : 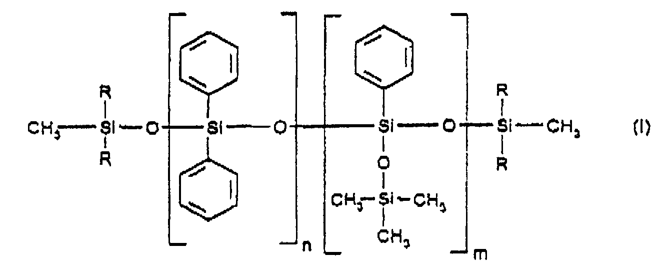 in which
. R is a C₁-C₃₀ alkyl radical, an aryl radical or an aralkyl radical,
. n is an integer between 0 and 100,
. m is an integer between 0 and 100, with the proviso that the sum m+n is between 1 and 100,
- (ii) poly-α-olefins of polybutene or polydecene type, and
- (iii) mixtures thereof.

19. Composition according to any one of Claims 14 to 18, characterized in that the binder is present at a content ranging from 1% to 30%, and preferably from 3% to 25%, by weight relative to the total weight of the composition.

20. Composition according to any one of Claims 14 to 19, characterized in that the pulverulent compounds are present at a content ranging from 70% to 99% by weight relative to the total weight of the composition.

21. Composition according to any one of Claims 14 to 20, characterized in that it is in the form of a blusher, an eyeshadow, a foundation or a body powder.
